# EUROPEAN PATENT APPLICATION

(11) **EP 1 561 454 A1**
(43) Date of publication of application: **10.08.2005**
(21) Application number: 05250241.6
(22) Date of filing: 19.01.2005
(51) Int. Cl.: A61K 7/16

(54) **Two-phase compositions containing alcohol**

(30) Priority: 20.01.2004 US 760933
(71) Applicant: Johnson & Johnson Consumer Companies, Inc., Skillman, New Jersey 08558 (US)
(72) Inventor: Jampani, Hanuman B., Flemington, NJ 08822 (US); Mignone, Pamela, Princeton, NJ 08540 (US)
(74) Representative: Mercer, Christopher Paul

(57) **Abstract**

The present invention relates to a two-phase composition that provides anti-microbial action and desorbs bacteria from solid surfaces and from living tissues, and in particular breath freshening and plaque removal. The composition provides fast and persistent anti-microbial activity and includes a hydro-alcoholic phase, an oil phase, and a cationic surface active agent.

## Description

### FIELD OF THE INVENTION

The present invention relates to a two-phase composition. One phase of the composition is hydro-alcoholic, and the other phase is an oil phase. When utilized as a mouth rinse, the composition desorbs bacteria from the teeth and other surfaces of the mouth and provides anti-microbial properties to the mouth.

The primary use for the composition of the present invention is in oral hygiene. Compositions of the invention can also be used in entirely different applications, where anti-microbial and bacterial desorption actions are desired.

### BACKGROUND OF THE INVENTION

Tooth decay and periodontal disease are due to bacterial accumulations on the surfaces of the teeth in the form of a macroscopic layer generally referred to as dental plaque. Dental plaque firmly adheres to the surface of teeth, and is composed of about 70% bacteria, about 20% polysaccharides produced by the bacteria and about 10% food remains. It is generally known that acids stored in dental plaque decalcify enamel, causing dental caries. The generation of dental caries is also linked to the presence of certain types of bacteria on the surfaces of teeth. Halitosis, generally referred to as bad breath, has been attributed to the presence and activity of bacteria in the oral cavity. Swollen gums, generally referred to as gingivitis, occurs when the bacteria in dental plaque causes the gums to become inflamed. In the mildest form of gingivitis the gums redden, swell and bleed easily. Accordingly, decreasing the amount of bacteria in the mouth in a fast and efficient way is desired in order to maintain fresh breath for a longer period of time and to prevent dental caries and gingivitis.

Mouth rinses are commonly utilized to freshen the breath and kill bacteria. Alcohol is typically utilized as the antimicrobial agent in a mouth rinse. However, alcohol can be damaging or irritating to oral tissues. If alcohol were to be used, it would be desirable to use lower levels of alcohol.

The desire for a liquid composition that offers long-lasting fresh breath in a mouth rinse without having oral tissue damage or irritation due to the presence of alcohol has led to the development of two-phase liquid compositions.

U.S. Patent No. 6,465,521, Rosenberg discloses a composition for desorbing bacteria from surfaces of the teeth. Rosenberg's invention uses a two-phase preparation of oil and water, which upon shaking forms a temporary oil-in-water emulsion. Unlike the present invention, Rosenberg's invention does not include a hydro-alcoholic phase that offers faster and more efficient anti-microbial activity with desired plaque desorption features.

### SUMMARY OF THE INVENTION

In accordance with this invention, there is provided a two-phase composition which upon shaking forms an emulsion, comprising:
a.) a hydro-alcholic phase; b.) an oil phase having a Hildebrand solubility parameter of from about 1 to about 7.5; and c.) a cationic surface active agent.

The invention will be more fully understood and further advantages will become apparent when reference is made to the following detailed description.

### DETAILED DESCRIPTION OF PREFERRED EMBODIMENTS

The two-phase composition of the present invention includes from about 50% to about 98% by weight, based on the total weight of the composition, of a hydro-alcohol phase. As used herein, hydro-alcohol phase means a mixture comprising water and ethyl alcohol. The amount of ethyl alcohol in the two-phase composition may range from about 2% to about 50%, preferably from about 5% to about 20%, more preferably from about 8% to about 12%, by weight, based on the total weight of the composition. The ethanol used in the practice of the present invention must be of a grade that is safe for oral use.

The amount of water in the two-phase composition may range from about 48% to about 96%, preferably from about 60% to about 95%, more preferably from about 80% to about 90%, by weight, based on the total weight of the composition.

The two-phase composition of the present invention further includes an oil phase having a Hildebrand solubility parameter of from about 1 to about 7.5. The amount of the oil phase may range from about 2% to about 50%, preferably from about 5% to about 30%, more preferably from about 10% to about 20%, by weight, based on the total weight of the composition. Suitable oils for use in the oil phase of the present invention include, but are not limited to, olive oil, corn oil, coconut oil, soybean oil, safflower oil, mineral oil, grapeseed oil, canola oil, sesame seed oil, cottonseed oil, polydecene and mixtures thereof. Other materials that are not soluble in water, including, but not limited to, lower alkyl esters of longer chain fatty acids, e.g., isopropyl palmitate, isopropyl myristate and mixtures thereof may be included in the oil phase, as long as the Hildebrand solubility parameter for the entire oil phase is from about 1 to about 7.5.

The compositions of the present invention also include at least one cationic surface active agent. The amount of cationic surface active agent may range from about 0.001% to about 5%, preferably from about 0.01% to about 0.1% by weight, based on the total weight of the composition. Suitable cationic surface active agents include, but are not limited to, pyridinium-based cationic surface-active molecules, such as cetylpyridinium chloride and laurylpyridinium chloride; chlorhexidine, chlorhexidine diacetate, chlorhexidine digluconate, and chlorhexidine dihydrochloride; monalkyl quaternary ammonium compounds, such as benzalkonium chloride, cetalkonium chloride, cetalkonium bromide, lauralkonium chloride, lauralkonium bromide, soytrimonium chloride, and polyethylene glycol-5- stearyl ammonium lactate; dialkyl quaternary ammonium compounds, such as dilauryl dimonium chloride, dicetyl dimonium chloride, dicetyl dimonium bromide, desqualinium chloride, and soyamido propyl benzyldimonium chloride; quatemiums, such as Quatemium 15 and polyquatemiums; amine fluorides; cationic polysaccharides, such as chitosan and its derivatives; and cationic polypeptides, such as poly L-lysine, poly D-lysine, and lysozyme.

Two-phase compositions are taught in United States Patent No. 6,465,521. The two-phase compositions of the '521 patent and of the present invention form an emulsion upon shaking.

The emulsion formed by shaking the two-phase composition of the present invention is swished around in the mouth of the user, thereby removing plaque from the teeth and killing bacteria in the mouth. The emulsion separates (or "breaks") back into a two-phase composition relatively quickly. The time it takes for the emulsion to break depends on the components of the two-phase composition and relative amounts thereof. Generally, the emulsion breaks within from about 30 seconds to about 30 minutes after shaking or agitation has stopped.

As is known in the art, commercially available colorants, flavorants, thickeners, and preservatives may be included in either or both of the phases of the two-phase compositions of the present invention in amounts known to those of ordinary skill in the art.

The compositions of the present invention may further include surfactants.

Suitable surfactants include nonionic and amphoteric surfactants.

Nonlimiting examples of nonionic surfactants include those selected from the group consisting of alkyl glucosides, alkyl polyglucosides, polyhydroxy fatty acid amides, alkoxylated fatty acid esters, sucrose esters, amine oxides, and mixtures thereof. Specific examples include, but are not limited to, nonionic surfactants selected from the group consisting of C8-C14 glucose amides, C8- C14 alkyl polyglucosides, sucrose cocoate, sucrose laurate, lauramine oxide, cocoamine oxide, and mixtures thereof.

Nonlimiting examples of amphoteric surfactants (which also includes zwitterionic surfactants) are those selected from the group consisting of betaines, sultaines, hydroxysultaines, alkyliminoacetates, iminodialkanoates, aminoalkanoates, and mixtures thereof.

Nonlimiting examples amphoteric surfactants useful in the practice of the present invention include disodium lauroamphodiacetate, sodium lauroamphoacetate, cetyl dimethyl betaine, cocoamidopropyl betaine, cocoamidopropyl hydroxy sultaine, and mixtures thereof.

The compositions of the present invention preferably further include lactic acid. The amount of lactic acid may range from about 0.01 percent to about 5 percent, preferably from about 0.05 percent to about 1 percent, more preferably from about 0.05 percent to about 0.5 percent by weight, based on the total weight of the composition.

The pH of the compositions of the present invention may affect the antimicrobial activity. The pH may range from 3.5 to 7.5, preferably from 3.7 to 4.3. The pH of the compositions of the present invention may be adjusted with any known acid. Suitable acids include, but are not limited to, benzoic acid, citric acid and the like. Citric acid is preferred. As is known in the art, the composition may be buffered to remain in the desired pH range.

Several Examples are set forth below. The claims should not be considered as being limited to the details thereof.

### Example 1 - Two-phase Compostion without Ethanol

The materials listed in Table 1 below were combined to provide a two-phase mouthwash composition without ethanol, similar to the Rosenberg '521 patent.

**Table 1**

| | |
|---|---|
| Water | 84.1221 |
| Ethanol | 0 |
| Sorbitol | 0.5 |
| Sodium Saccharin | 0.15 |
| Monosodium Phosphate | 0.05 |
| Cetylpyridinium Chloride | 0.05 |
| Sodium Methylparaben | 0.05 |
| Disodium EDTA | 0.05 |
| Citric Acid | 0.0167 |
| FD&C Blue #1 | 0.00018 |
| D&C Yellow 10 | 0.00105 |
| Isopropyl Myristate | 14.81 |
| Mint Flavor | 0.2 |
| Total | 100.00 |

The Hildebrand Solubility Parameter ("HSP") of the oil phase of Example 1 (the only component of which is isopropyl myristate) was 7.78.

The formulations of Example 1 and the following Examples herein were evaluated following the standard time-kill test protocol described below. Formulations that demonstrated at least a 2-log reduction of bacteria in 30 seconds with about 10% alcohol were considered to be acceptable.

### MATERIALS

Fresh broth culture of *Fusobacterium nucleatum,* ATCC 25586.

| | |
|---|---|
| Deionized water (sterile). | 9 mL Trypticase Soy broth + 25% Capitol IV Broth. |
| Bulk agar prepared: Brain-Heart Infusion agar. | |
| Sterile disposable Petri dishes (100 x 15 mm). | |
| Sterile disposable pipets. | Waterbath (40°C ± 2°C). |
| Stopwatch (or equivalent). | Vortex mixer. |
| Incubator at 35°C (± 2°C). | 0.85% Saline (or equivalent). |
| Anaerobic Chamber. | |

A sterile deionized water control was prepared separately and tested for test validation purposes. Undiluted test samples were vortexed to combine the two phases in the mouth rinse prior to inoculation of the test organisms. Test and control samples were inoculated with inoculum suspensions to yield 10⁵-10⁶ CFU/mL at a ratio of 1:100 (V/W). The test samples were vortexed for 30 seconds, then 1 mL aliquots were transferred from the test and control samples to 9 mL of Broth to neutralize antimicrobial activity (i.e. 1:10 dilution in neutralized broth). Serial dilutions were prepared and the total plate count of each aliquot was determined. Pour plating for a total count was conducted using an agar and incubation temperature which readily supported test microorganism growth. Total plate counts of the test formulation and control sample were compared at the 30 second time interval to determine the microbiocidal activity of the test formulation. Results are reported as microbial log reduction exhibited by the test formulation as compared to the control sample.

The Example 1 formulation exhibited 0.8-log reduction (microbial kill) at 60 seconds. In a 30 second time kill test, the example 1 formulation exhibited a 0.0-log reduction. In an attempt to achieve rapid kill, alcohol based samples were prepared at two different concentrations as shown in Example 2 and 3 below.

### Example 2 - Two-phase Composition with 5% Ethanol

The materials listed in Table 2 below were combined to form the two-phase mouthwash composition of Example 2.

**Table 2**

| | |
|---|---|
| Water | 79.1221 |
| Ethanol | 5 |
| Sorbitol | 0.5 |
| Sodium Saccharin | 0.15 |
| Monosodium Phosphate | 0.05 |
| Cetylpyridinium Chloride | 0.05 |
| Sodium Methylparaben | 0.05 |
| Disodium EDTA | 0.05 |
| Citric Acid | 0.0167 |
| FD&C Blue #1 | 0.00018 |
| D&C Yellow 10 | 0.00105 |
| Peppermint Oil #35 | 1.5 |
| Isopropyl Myristate | 13.51 |
| Total | 100.00 |
| HSP = 7.78 | |

### Example 3 - Two-phase Composition with 10% Ethanol

The materials listed in Table 3 below were combined to form the two-phase mouthwash composition of Example 3.

**Table 3**

| | |
|---|---|
| Water | 74.1221 |
| Ethanol | 10 |
| Sorbitol | 0.5 |
| Sodium Saccharin | 0.15 |
| Monosodium Phosphate | 0.05 |
| Cetylpyridinium Chloride | 0.05 |
| Sodium Methylparaben | 0.05 |
| Disodium EDTA | 0.05 |
| Citric Acid | 0.0167 |
| FD&C Blue #1 | 0.00018 |
| D&C Yellow 10 | 0.00105 |
| Isopropyl Myristate | 13.51 |
| Peppermint Oil | 1.5 |
| Total | 100.00 |
| HSP = 7.78 | |

### Example 4 - Two-phase Composition with 14% Ethanol

The materials listed in Table 4 below were combined to provide the two-phase mouthwash composition of Example 4.

**Table 4**

| | |
|---|---|
| Water | 70.1221 |
| Ethanol | 14 |
| Sorbitol | 0.5 |
| Sodium Saccharin | 0.15 |
| Monosodium Phosphate | 0.05 |
| Cetylpyridinium Chloride | 0.05 |
| Sodium Methylparaben | 0.05 |
| Disodium EDTA | 0.05 |
| Citric Acid | 0.0167 |
| FD&C Blue #1 | 0.00018 |
| D&C Yellow 10 | 0.00105 |
| Isopropyl Myristate | 14.87 |
| SymriseMint 825555 | 0.14 |
| Total | 100.00 |
| HSP = 7.78 | |

The above composition provided an approximately 3.6 log reduction in bacteria. Though the two-phase formulations of Examples 2 and 3 contained ethanol at 5% and 10% respectively, they did not demonstrate a 2-log reduction in bacteria. Since isopropyl myristate is soluble in ethanol, it was hypothesized that possibly the alcohol was solubilizing the oil phase components, and thus not sufficiently getting exposed to microorganisms to achieve immediate antimicrobial activity. However, a composition with 14% alcohol may burn or irritate the oral mucosa; therefore it was desired to have the same micro kill efficacy at lower alcohol levels.

In order to test the hypothesis, an oil less soluble with ethanol, mineral oil, was substituted for isopropyl myristate and the alcohol level was reduced to 10%. The resulting two-phase composition was tested for its antimicrobial efficacy. See Example 5.

### Example 5 - Two-phase Composition with 15% Mineral Oil and 10% Ethanol

The materials listed in Table 5 below were combined to provide the two-phase mouthwash composition of Example 5.

**Table 5**

| | |
|---|---|
| Water | 74.114 |
| Ethanol | 10 |
| Sorbitol | 0.5 |
| Sodium Saccharin | 0.15 |
| Cetylpyridinium Chloride | 0.05 |
| Sodium Methylparaben | 0.05 |
| Disodium EDTA | 0.05 |
| Citric Acid | 0.07 |
| Benzoic Acid | 0.005 |
| FD&C Blue #1 | 0.0001 |
| D&C Yellow 10 | 0.00105 |
| Mineral Oil #35 | 15.01 |
| Total | 100.00 |
| HSP = 7.09 | |

The two-phase composition of Example 5 demonstrated a 4.2 log reduction in bacteria.

### Example 6 - Two-phase Composition with 15% Oil blend and 10% Ethanol

The two-phase composition of this Example 6 was the same as that of Example 5 except the 15.01 parts of mineral oil in Example 5 was replaced by an equal amount of a 50/50 (wt%) blend of isopropyl myristate and mineral oil. The HSP of the oil phase of this Example 6 was 7.44. Upon testing in the manner set forth for the earlier Examples, the two-phase composition of Example 6 demonstrated a 4.1 log reduction in bacteria.

**Table 6**

| | |
|---|---|
| Water | 73.40385 |
| Ethanol | 10 |
| Sorbitol | 0.5 |
| Sodium Saccharin | 0.15 |
| Cetylpyridinium Chloride | 0.05 |
| Disodium EDTA | 0.1 |
| Citric Acid | 0.04 |
| Benzoic Acid | 0.005 |
| FD&C Blue #1 | 0.0001 |
| D&C Yellow 10 | 0.00105 |
| Mineral Oil #35 | 7.5 |
| Isopropyl Myristate | 7.5 |
| Flavor | 0.75 |
| Total | 100.00 |
| HSP=7.44 | |

Table 7 shown below summarizes each example by showing the log-reduction of each formulation, as well as the percentage of alcohol used.

**Table 7**

| Example # | Alcohol % | HSP | 30 Second Log Reduction |
|---|---|---|---|
| 1 | 0 | 7.78 | 0.0 |
| 2 | 5 | 7.78 | 0.9 |
| 3 | 10 | 7.78 | 0.9 |
| 4 | 14 | 7.78 | 3.6 |
| 5 | 10 | 7.09 | 4.2 |
| 6 | 10 | 7.44 | 4.1 |

### Example 7 - pH Studies

Three formulations at three pHs (3.5, 4, 4.5) were investigated. The first formulation (A) contained only water and lactic acid in the aqueous phase. The second formulation (B) was a two-phase mouthwash formulation with lactic acid. The third formulation (C ) was a two-phase mouthwash formulation without lactic acid.

The base formulation from which formulations A, B, and C were prepared is listed in Table 8 below.

**Table 8**

| | Water + Lactic Acid (A) | Mouthwash with Lactic Acid (B) | Mouthwash without Lactic Acid (C) |
|---|---|---|---|
| Water | 99.9 | 74.089 | 74.184 |
| Ethanol | - | 10 | 10 |
| Sorbitol | - | 0.5 | 0.5 |
| Sodium Saccharin | - | 0.15 | 0.15 |
| CPC | - | 0.05 | 0.05 |
| EDTA | - | 0.05 | 0.05 |
| Sodium Methylparaben | - | 0.05 | 0.05 |
| Lactic Acid | 0.1 | 0.1 | - |
| FD&C Blue #1 | - | 0.0001 | 0.0001 |
| D&C Yellow 10 | - | 0.00105 | 0.00105 |
| | | | |
| Mineral Oil #35 | 15.01 | 15.01 | 15.01 |

All formulations were 2-phase mouthwashes, where the water/oil ratio was 85/15. The pH for the A series was adjusted up with Sodium Phosphate Dibasic. The pH for the B series was adjusted up or down using Sodium Phosphate Dibasic or citric acid. The pH for the C series was adjusted down using citric acid.

Microbiology tests were done with *Streptococcus mutans* ATCC 25175. The diluent was TSB + CAP IV. The media was brain heart, incubation 37°C aerobically for 48-72 hours minimum. The control count for *S*. *mutans* was 6.2 x 10⁶. Each sample was run in duplicate. The test method was similar to that described above:

| | |
|---|---|
| Deionized water (sterile). | 9 mL Trypticase Soy broth + 25% Capitol IV Broth. |
| Bulk agar prepared: Brain-Heart Infusion agar. | |
| Sterile disposable Petri dishes (100 x 15 mm). | |
| Sterile disposable pipets. | Stopwatch (or equivalent). |
| Vortex mixer. | Incubator at 35°C (± 2°C). |
| 0.85% Saline (or equivalent). | |

A sterile deionized water control was prepared separately and tested for test validation purposes. Test samples were vortexed to combine the two phases in the mouth rinse prior to inoculation of the test organisms. Test and control samples were inoculated with inoculum suspensions to yield 10⁵-10⁶ CFU/mL at a ratio of 1:100 (V/W). The test samples were vortexed for 30 seconds, then 1 mL aliquots were transferred from the test and control samples to 9 mL of Broth to neutralize antimicrobial activity (ie. 1:10 dilution in neutralized broth). Serial dilutions were prepared and the total plate count of each aliquot was determined. Pour plating for a total count was conducted using an agar and incubation temperature which readily supported test microorganism growth. Total plate counts of the test formulation and control sample were compared at the 30 and 60 second time interval to determine the microbiocidal activity of the test formulation. Results are reported as microbial log reduction exhibited by the test formulation as compared to the control sample.

### 60 Second Data

| Water + Lactic Acid (A) | | | | | | |
|---|---|---|---|---|---|---|
| pH | cfu/ml | | Log reduction | | | |
| | Replicate #1 | Replicate #2 | Replicate #1 | Replicate #2 | AVG | STD |
| 4.44 | >3e5 | >3e5 | 0 | 0 | 0 | 0 |
| 3.98 | >3e5 | >3e5 | 0 | 0 | 0 | 0 |
| 3.49 | >3e5 | >3e5 | 0 | 0 | 0 | 0 |

| Mouthwash with Lactic Acid (B) | | | | | | |
|---|---|---|---|---|---|---|
| pH | cfu/ml | | Log reduction | | | |
| | Replicate #1 | Replicate #2 | Replicate #1 | Replicate #2 | AVG | STD |
| 4.05 | 30 | 30 | 5.3 | 5.3 | 5.3 | 0 |
| 4.43 | 130 | 140 | 4.7 | 4.6 | 4.7 | 0.02 |
| 3.57 | 10 | 20 | 5.8 | 5.5 | 5.6 | 0.21 |

| Mouthwash without Lactic Acid (C) | | | | | | |
|---|---|---|---|---|---|---|
| pH | cfu/ml | | Log reduction | | | |
| | Replicate #1 | Replicate #2 | Replicate #1 | Replicate #2 | AVG | STD |
| 4.50 | 40 | 20 | 5.2 | 5.5 | 5.3 | 0.21 |
| 3.98 | 460 | 570 | 4.1 | 4.0 | 4.1 | 0.07 |
| 3.55 | 10 | 10 | 5.8 | 5.8 | 5.8 | 0.00 |

### 30 Second Data

| Water + Lactic Acid (A) | | | | | | |
|---|---|---|---|---|---|---|
| pH | cfu/ml | | Log reduction | | | |
| | Replicate #1 | Replicate #2 | Replicate #1 | Replicate #2 | AVG | STD |
| 4.44 | >3e5 | >3e5 | 0 | 0 | 0 | 0 |
| 3.98 | >3e5 | >3e5 | 0 | 0 | 0 | 0 |
| 3.49 | >3e5 | >3e5 | 0 | 0 | 0 | 0 |

| Mouthwash with Lactic Acid (B) | | | | | | |
|---|---|---|---|---|---|---|
| pH | cfu/ml | | Log reduction | | | |
| | Replicate #1 | Replicate #2 | Replicate #1 | Replicate #2 | AVG | STD |
| 4.05 | 200 | 160 | 4.5 | 4.6 | 4.54 | 0.07 |
| 4.43 | 1700 | 1670 | 3.6 | 3.6 | 3.57 | 0.01 |
| 3.57 | 420 | 430 | 4.2 | 4.2 | 4.16 | 0.01 |

| Mouthwash without Lactic Acid (C) | | | | | | |
|---|---|---|---|---|---|---|
| | cfu/ml | | Log reduction | | | |
| pH | Replicate #1 | Replicate #2 | Replicate #1 | Replicate #2 | AVG | STD |
| 4.50 | 260 | 280 | 4.4 | 4.3 | 4.36 | 0.02 |
| 3.98 | 310 | 300 | 4.3 | 4.3 | 4.31 | 0.01 |
| 3.55 | 160 | 130 | 4.6 | 4.7 | 4.63 | 0.06 |

The lactic acid in water samples did not provide microbial kill at any pH level that was tested. The data for the mouthwash with lactic acid and the mouthwash without lactic acid was graphed. A linear regression and a polynomial fit were also performed. Based on the data, it was determined that mouthwash with lactic acid provided enhanced microbial kill at a pH from 3.7 to 4.3 when compared to the same mouthwash without lactic acid.

## Claims

1. A two-phase composition which, upon shaking, forms an emulsion, comprising:
a.) a hydro-alcholic phase;
b.) an oil phase having a Hildebrand solubility parameter of from 1 to 7.5; and
c.) a cationic surface active agent.

2. The composition of claim 1 wherein the hydro-alcoholic phase comprises from 2% to 50% by weight alcohol and from 48% to 96% by weight water, based on the total weight of the composition.

3. The composition according to claim 2 wherein the alcohol is ethanol.

4. The composition according to any one of claims 1 to 3 wherein the amount of the oil phase ranges from 2% to 50% by weight, based on the total weight of the composition.

5. The composition according to claim 4 wherein the oil phase comprises olive oil, corn oil, coconut oil, soybean oil, safflower oil, mineral oil, grapeseed oil, canola oil, sesame seed oil, cottonseed oil, polydecene or a mixture thereof.

6. The composition according to any one of claims 1 to 5 wherein the amount of the cationic surface active agent ranges from 0.001% to 5% by weight, based on the total weight of the composition.

7. The composition according to claim 6 wherein the cationic surface active agent is cetylpyridinium chloride, laurylpyridinium chloride, chlorhexidine, chlorhexidine diacetate, chlorhexidine digluconate, chlorhexidine dihydrochloride, benzalkonium chloride, cetalkonium chloride, cetalkonium bromide, lauralkonium chloride, lauralkonium bromide, soytrimonium chloride, polyethylene glycol-5 stearyl ammonium lactate, dilauryl dimonium chloride, dicetyl dimonium chloride, dicetyl dimonium bromide, dequalinium chloride, soyamido propyl benzyldimonium chloride, quatemium 15, a polyquatemium, an amine fluoride, chitosan, poly L-lysine, poly D-lysine, lysozyme or a combination thereof.

8. The composition according to claim 7 wherein the cationic surface active agent is cetylpyridinium chloride.

9. The composition according to any one of claims 1 to 8 further comprising lactic acid.

10. The composition according to claim 9 wherein the composition has a pH of from 3.7 to 4.3.
